(19)

Europäisches
Patentamt
European
Patent Office
Office européen
des brevets

(11) **EP 4 299 745 A1**

(12) **EUROPEAN PATENT APPLICATION**
published in accordance with Art. 153(4) EPC

(43) Date of publication:
**03.01.2024 Bulletin 2024/01**

(21) Application number: **22759586.5**

(22) Date of filing: **21.02.2022**

(51) International Patent Classification (IPC):
*C12N 15/13* (2006.01)   *A61K 39/395* (2006.01)
*A61P 31/14* (2006.01)   *A61P 43/00* (2006.01)
*C07K 16/10* (2006.01)   *C07K 19/00* (2006.01)
*G01N 33/569* (2006.01)

(52) Cooperative Patent Classification (CPC):
**A61K 39/395; A61P 31/14; A61P 43/00;
C07K 16/00; C07K 16/10; C07K 19/00;
G01N 33/569**

(86) International application number:
**PCT/JP2022/006996**

(87) International publication number:
**WO 2022/181550 (01.09.2022 Gazette 2022/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.02.2021   JP 2021029050**

(71) Applicants:
• **Kao Corporation
Chuo-ku
Tokyo 103-8210 (JP)**
• **Epsilon Molecular Engineering Inc.
Saitama City, Saitama 338-8570 (JP)**

(72) Inventors:
• **INAURA, Shunsuke
Haga-gun, Tochigi 321-3497 (JP)**
• **MATSUMURA, Yuta
Haga-gun, Tochigi 321-3497 (JP)**
• **TOJO, Takuto
Wakayama-shi, Wakayama 640-8580 (JP)**
• **NAGAMI, Atsushi
Haga-gun, Tochigi 321-3497 (JP)**

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54) **ANTI-SARS-COV-2 ANTIBODY**

(57)    Provided is an antibody that binds to the N-protein of SARS-CoV-2, and a method for utilizing the antibody. An antibody that binds to SARS-CoV-2, having one or more structural domains comprising CDRs shown in the following (a) or (b): (a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or (b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

EP 4 299 745 A1

# Figure 1

**Description**

Field of the Invention

The present invention relates to an antibody that binds to the nucleocapsid protein of SARS-CoV-2.

Background of the Invention

SARS coronavirus-2 (Severe acute respiratory syndrome coronavirus 2; SARS-CoV-2) belongs to the genus *Betacoronavirus,* as with SARS coronavirus (SARS-CoV) and MERS coronavirus (MERS-CoV), and is a SARS-related coronavirus which causes acute respiratory disease (COVID-19). An outbreak was first identified near Wuhan City, Hubei Province, China in 2019, and this coronavirus has since caused a global outbreak (pandemic) of COVID-19.

SARS-CoV-2 is a single-stranded positive-sense RNA virus with a viral genome of about 29,903 bases. Virus particles (virions) have a size of from about 50 to 200 nm. Like common coronaviruses, this virus is composed of four proteins known as spike, nucleocapsid, integral membrane, and envelope proteins, as well as RNA. Of these, the nucleocapsid protein binds to RNA to form a nucleocapsid, and the lipid-binding spike protein, integral membrane protein, and envelope protein surround the nucleocapsid to form an envelope (Non-Patent Literatures 2 and 3).

The nucleocapsid protein (hereinafter, N-protein) of SARS-CoV-2 is a protein which binds to the RNA genome of SARS-CoV-2. This protein is reported to be involved in virus particle formation, such as folding of the viral genome, assembly of viral proteins, and viral budding, as well as to have the function of suppressing the response of host cells (Non-Patent Literature 4).

The virological diagnosis of COVID-19 is mainly based on the detection of SARS-CoV-2 gene by gene amplification (PCR). PCR tests are considered to be the most sensitive viral test available at present. Other detection methods include antigen tests which detect fragments of viral surface proteins (e.g., spike protein). For antigen tests, detection kits based on immunochromatography and ELISA are already commercially available. Although their detection sensitivity is generally inferior to that of PCR tests, the detection kits are superior in terms of the time required and the ease of use.

Virus testing requires a test method without false positives and false negatives. For false positives, a measurement principle with high specificity for the target molecules is important, and for false negatives, the detection sensitivity of the test method is important. Part of the virus structure is present at a low concentration in body fluids (saliva, pharyngeal mucus, and the like) and sewage derived from SARS-CoV-2-infected persons. Existing PCR and antigen test methods may not be sufficient to detect these viruses. Therefore, while PCR tests and antigen tests for SARS-CoV-2 have already become general-purpose technologies, there is a strong demand for technological development to detect SARS-CoV-2 more specifically and with higher sensitivity.

The N-protein of SARS-CoV-2 is reported to be one of the most abundant proteins which make up the virus (Non-Patent Literature 4). Since the number of N-proteins is much larger than that of the surface-exposed spike proteins of SARS-CoV-2, the N-proteins are preferred as target molecules in antigen tests for SARS-CoV-2, especially in terms of detection sensitivity. Further, the N-protein is present bound to the SARS-CoV-2 RNA genome as a nucleocapsid. Therefore, for example, if an antibody which can selectively and strongly bind to the N-protein as a target can be developed, and a SARS-CoV-2 N-protein-RNA genome complex can be concentrated, it will be possible to detect SARS-CoV-2 with higher sensitivity in PCR tests. Similarly, by combining the step of concentrating the N-protein with antigen tests, it will also be possible to increase the sensitivity of the antigen tests for SARS-CoV-2.

The N-protein of SARS-CoV-2 is one of the proteins mainly expressed in infected cells and exhibits strong immunogenicity. It has been reported that ELISA for the N-protein of SARS-CoV, which was prevalent from November 2002, was able to diagnose patients who were suspected to be in the early stages of infection with high specificity (Non-Patent Literature 5).

A normal antibody (e.g., an IgG antibody) binds to an antigen in its two variable regions of heavy and light chains; however, the heavy-chain antibody found in camelid serum shows binding activity and antigen specificity in only one variable region. The variable region of this antibody is called VHH (variable domain of heavy chain of heavy-chain antibody), and is considered to have the lowest molecular weight (about 1/10 of a normal antibody (e.g., an IgG antibody)) as an immunoglobulin fragment which can bind to an antigen (Non-Patent Literature 6). Functional features of VHH antibodies include 1) having a binding activity equal to or greater than that of normal antibodies, 2) able to enter areas which are not accessible by normal antibodies, 3) high facilitation of formation and regeneration of physiological structures, and 4) having a very simple structure because they are single-stranded peptides. In particular, for the reason of 3), VHH antibodies are extremely stable against heat and pressure, and it is possible to mass-produce functional antibodies in prokaryotes *(Escherichia coli, Bacillus subtilis,* Corynebacterium, yeast, and the like), which has been difficult in the past. Further, for the reason of 4), VHH antibodies are characterized in that it is easy to modify their function by protein engineering or chemical modification, and that it is easy to produce antibody-drug conjugates (ADCs).

Thus, VHH antibodies have many advantages in terms of properties and production compared to normal antibodies (e.g., IgG antibodies). Therefore, detection technology using VHH antibodies targeting the N-protein of SARS-CoV-2 is expected to be more advantageous in terms of performance and price than conventional technology using IgG antibodies.

[Non-Patent Literature 1] A pneumonia outbreak associated with a new coronavirus of probable bat origin. Nature. 2020 Mar; 579 (7798): 270-273.

[Non-Patent Literature 2] A new coronavirus associated with human respiratory disease in China. Nature. 2020 Mar; 579 (7798): 265-269.

[Non-Patent Literature 3] The Coronavirus Nucleocapsid Is a Multifunctional Protein. Viruses. Volume 6, Issue 8, August 2014, Pages 2991-3018.

[Non-Patent Literature 4] The SARS-CoV nucleocapsid protein: A protein with multifarious activities. Infection, Genetics and Evolution. Volume 8, July 2008, Pages 397-405.

[Non-Patent Literature 5] Diagnosis of Severe Acute Respiratory Syndrome (SARS) by Detection of SARS Coronavirus Nucleocapsid Antibodies in an Antigen-Capturing Enzyme-Linked Immunosorbent Assay. Journal of Clinical Microbiology. Volume 41, No. 12, December 2003, Pages 5781-5782.

[Non-Patent Literature 6] The Therapeutic Potential of Nanobodies. BioDrugs. Volume 34, November 2019, Pages 11-26.

Summary of the Invention

The present invention relates to the following 1) to 8) .

1) An antibody that binds to SARS-CoV-2, having one or more structural domains comprising CDRs shown in the following (a) or (b):

(a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or

(b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

2) A nucleic acid encoding the antibody of 1).

3) A method for detecting SARS-CoV-2 in a sample, comprising a step of contacting an antibody that binds to SARS-CoV-2 with a test sample, the antibody having one or more structural domains comprising CDRs shown in the following (a) or (b):

(a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or

(b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

4) A kit for detecting SARS-CoV-2, comprising the antibody of 1).

5) A medicament comprising the antibody of 1).

6) Use of the antibody of 1), for producing a medicament.

7) The antibody of 1), for use as a medicament.

8) A method for preventing or treating SARS-CoV-2 infection, comprising administering the antibody of 1) to a subject in need thereof.

Brief Description of the Drawings

[Figure 1] Figure 1 shows alignments of the amino acid sequences of CoVHH-N1 and CoVHH-N1 variants selected by selection. * indicates the conserved amino acid. In comparison with the amino acid sequence of CoVHH-N1, the position where the amino acid is substituted is surrounded by a box for indication.

[Figure 2] Figure 2 shows the results of SDS-PAGE of synthesized VHHs. The arrows indicate target protein bands.

[Figure 3] Figure 3 shows the measurement of the binding activity of CoVHH-N1 to the N-protein by a surface plasmon resonance method. The black line is a binding dissociation curve based on the measured raw data, and the gray line is a binding dissociation curve after fitting.

[Figure 4] Figure 4 shows the measurement of the binding activity of CoVHH-N2 to the N-protein by a surface plasmon resonance method. The black line is a binding dissociation curve based on the measured raw data, and the gray line is a binding dissociation curve after fitting.

[Figure 5] Figure 5 shows the evaluation of the binding specificity of CoVHH-N1 and CoVHH-N2 to the N-protein of coronavirus by an ELISA method.

[Figure 6] Figure 6 shows the evaluation of the binding specificity of CoVHH-N2 to the N-protein of SARS-CoV-2 variant coronavirus by an ELISA method.

[Figure 7] Figure 7 shows the evaluation of the binding specificity of CoVHH-N2 to the N-protein of SARS-CoV-2 variant coronavirus by an ELISA method.

[Figure 8] Figure 8 shows the evaluation of the binding specificity of CoVHH-N1 and CoVHH-N2 to the N-protein of SARS-CoV-2 omicron coronavirus by an ELISA method.

Detailed Description of the Invention

The present invention relates to provision of an antibody that binds to the N-protein of SARS-CoV-2, and a method for utilizing the antibody.

As a result of studying to obtain a VHH antibody that binds to the N-protein of SARS-CoV-2, the present inventors succeeded in obtaining clones with high affinity for the N-protein of SARS-CoV-2 from a VHH antibody library containing CDRs 1 to 3 having a specific number of amino acids in constructs by screening by cDNA display.

According to the present invention, a high-affinityanti-SARS-CoV-2 N-protein antibody can be provided. The use of this antibody allows detection of SARS-CoV-2, that is, rapid detection of acute respiratory disease (COVID-19), which is SARS-CoV-2 infection.

The antibody that binds to SARS-CoV-2 according to the present invention (hereinafter referred to as "the antibody of the present invention") is an anti-SARS-CoV-2 antibody having one or more structural domains containing CDRs 1 to 3 shown in the following (a) or (b):

(a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or

(b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

SARS-CoV-2 is a SARS-related coronavirus which causes acute respiratory disease (COVID-19), and is a single-stranded positive-sense RNA virus with a viral genome of about 29,903 bases. The antibody of the present invention is an antibody that binds to SARS-CoV-2, and specifically an antibody that binds to the N-protein of SARS-CoV-2.

The structural domain of the antibody of the present invention has three CDRs, i.e., CDR1, CDR2, and CDR3.

CDRs (complementarity-determining regions) include a sequence-variable antigen recognition site or a random sequence region, and are also called hypervariable regions. In the structural domain of the antibody of the present invention, the three CDRs are present in the order of CDR1, CDR2, and CDR3 from the N-terminal side.

In an aspect of the antibody of the present invention, in the structural domain,

CDR1 consists of the amino acid sequence of SEQ ID NO: 1 or an amino acid sequence obtained by substituting one amino acid with another amino acid in the amino acid sequence,

CDR2 consists of the amino acid sequence of SEQ ID NO: 2 or an amino acid sequence obtained by substituting one amino acid with another amino acid in the amino acid sequence, and

CDR3 consists of the amino acid sequence of SEQ ID NO: 3 or an amino acid sequence obtained by substituting one amino acid with another amino acid in the amino acid sequence.

The amino acid sequence of SEQ ID NO: 1 is GFPFTKFWMY, the amino acid sequence of SEQ ID NO: 2 is FIDTTGTITN, and the amino acid sequence of CDR3 consisting of the amino acid sequence of SEQ ID NO: 3 is GPPGSTWWDFHS.

In another aspect of the antibody of the present invention, in the structural domain,

CDR1 consists of the amino acid sequence of SEQ ID NO: 4 or an amino acid sequence obtained by substituting one amino acid with another amino acid in the amino acid sequence,

CDR2 consists of the amino acid sequence of SEQ ID NO: 5 or an amino acid sequence obtained by substituting one amino acid with another amino acid in the amino acid sequence, and

CDR3 consists of the amino acid sequence of SEQ ID NO: 6 or an amino acid sequence obtained by substituting one amino acid with another amino acid in the amino acid sequence.

The amino acid sequence of SEQ ID NO: 4 is GFTFDDHAVG, the amino acid sequence of SEQ ID NO: 5 is AISGDGYTTV, and the amino acid sequence of CDR3 consisting of the amino acid sequence of SEQ ID NO: 6 is FRTDIVREY.

The CDR consisting of an amino acid sequence obtained by substituting one amino acid with another amino acid in any of the amino acid sequences of SEQ ID NOs: 1 to 6 refers to a CDR obtained by introducing variation into the CDR consisting of any of the amino acid sequences of SEQ ID NOs: 1 to 6. CDR1, CDR2, and CDR3 into which variation is introduced are also included in the CDRs of the antibody of the present invention as long as they have the binding affinity for SARS-CoV-2.

The position of the amino acid to be substituted is not limited, but is preferably, for example, position 1, 2, 4, 6, 7, 8, or 9 in CDR1 consisting of the amino acid sequence of SEQ ID NO: 1, position 1 or 10 in CDR2 consisting of the amino acid sequence of SEQ ID NO: 2, and position 1, 6, 9, or 12 in CDR3 consisting of the amino acid sequence of SEQ ID NO: 3.

The type of amino acid after substitution is not limited. Examples of substitution include substitution with amino acids having similar polarity and size. Other examples of substitution include substitution of a glycine residue with an arginine residue, substitution of a phenylalanine residue with a leucine residue or a cysteine residue, substitution of a lysine residue with an asparagine residue, substitution of a tryptophan residue with a cysteine residue, substitution of a methionine residue with a valine residue or an arginine residue, substitution of an asparagine residue with an aspartic acid residue, substitution of a glycine residue with a cysteine residue, substitution of a threonine residue with an alanine residue or a proline residue, substitution of a valine residue with an isoleucine residue, substitution of a histidine residue with an asparagine residue or an aspartic acid residue, and the like.

Examples of preferred combinations of the position of the amino acid to be substituted and the type of amino acid after substitution include:

in CDR1 consisting of the amino acid sequence of SEQ ID NO: 1, substitution of glycine residue at position 1 with an arginine residue, substitution of phenylalanine residue at position 2 with a leucine residue, substitution of phenylalanine residue at position 4 with a leucine residue, substitution of lysine residue at position 6 with an asparagine residue, substitution of phenylalanine residue at position 7 with a leucine residue or a cysteine residue, substitution of tryptophan residue at position 8 with a cysteine residue, and substitution of methionine residue position 9 with a valine residue or an arginine residue;

in CDR2 consisting of the amino acid sequence of SEQ ID NO: 2, substitution of phenylalanine residue at position 1 with a leucine residue and substitution of asparagine residue at position 10 with an aspartic acid residue; and

in CDR3 consisting of the amino acid sequence of SEQ ID NO: 3, substitution of glycine residue at position 1 with a cysteine residue, substitution of threonine residue at position 6 with an alanine residue or a proline residue, substitution of valine residue at position 9 with an isoleucine residue, and substitution of histidine residue at position 12 with an asparagine residue or an aspartic acid residue.

The binding affinity for SARS-CoV-2 can be evaluated by a method known to a person skilled in the art. Specifically, as shown in the Examples described later, the affinity can be evaluated by determining the equilibrium dissociation constant KD by a surface plasmon resonance method. The affinity can also be evaluated by, for example, ELISA with immobilized antigen, immunochromatography, isothermal titration calorimetry, biolayer interferometry, or the like.

The structural domain of the antibody of the present invention may have framework regions at both ends of CDR1, CDR2, and CDR3. The framework regions refer to variable regions of antibody molecules excluding complementarity-determining regions, and to highly conserved regions.

That is, in an aspect, the structural domain of the present invention has the first framework region (FR1), CDR1, the second framework region (FR2), CDR2, the third framework region (FR3), CDR3, and the fourth framework region (FR4) in this order.

The amino acid sequences of the framework regions in the structural domain include the following.

FR1: the amino acid sequence of SEQ ID NO: 7 or 8, or an amino acid sequence having 80% or more identity to the amino acid sequence

FR2: the amino acid sequence of SEQ ID NO: 9 or 10, or an amino acid sequence having 80% or more identity to the amino acid sequence

FR3: the amino acid sequence of SEQ ID NO: 11 or 12, or an amino acid sequence having 80% or more identity to the amino acid sequence

FR4: the amino acid sequence of SEQ ID NO: 13, or an amino acid sequence having 80% or more identity to the amino acid sequence

Framework regions consisting of an amino acid sequence having 80% or more identity to the amino acid sequences of SEQ ID Nos: 7 to 13 include framework regions consisting of an amino acid sequence preferably having 85% or more, more preferably 90% or more, more preferably 95% or more, more preferably 96% or more, more preferably 97% or more, more preferably 98% or more, and more preferably 99% or more identity.

The amino acid sequence identity as mentioned herein refers to the ratio (%) of the number of positions where the same amino acid residues are present in both of two aligned amino acid sequences to the number of full-length amino acid residues. The sequence identity can be calculated by analysis using, for example, BLAST (Basic Local Alignment Search Tool) of the National Center for Biotechnology Information (NCBI).

Of the antibodies of the present invention, an antibody having a structural domain (SEQ ID NO: 14) in which FR1, CDR1, FR2, CDR2, FR3, CDR3, and FR4 are linked in this order, and in which CDR1 consists of the amino acid sequence of SEQ ID NO: 1, CDR2 consists of the amino acid sequence of SEQ ID NO: 2, CDR3 consists of the amino acid sequence of SEQ ID NO: 3, FR1 consists of the amino acid sequence of SEQ ID NO: 7, FR2 consists of the amino acid sequence of SEQ ID NO: 9, FR3 consists of the amino acid sequence of SEQ ID NO: 11, and FR4 consists of the amino acid sequence of SEQ ID NO: 13, and an antibody having a structural domain (SEQ ID NO: 15) in which FR1, CDR1, FR2, CDR2, FR3, CDR3, and FR4 are linked in this order, and in which CDR1 consists of the amino acid sequence of SEQ ID NO: 4, CDR2 consists of the amino acid sequence of SEQ ID NO: 5, CDR3 consists of the amino acid sequence of SEQ ID NO: 6, FR1 consists of the amino acid sequence of SEQ ID NO: 8, FR2 consists of the amino acid sequence of SEQ ID NO: 10, FR3 consists of the amino acid sequence of SEQ ID NO: 12, and FR4 consists of the amino acid sequence of SEQ ID NO: 13, are clones with high affinity for SARS-CoV-2 (former: CoVHH-N1, latter: CoVHH-N2) each obtained by screening by cDNA display in the Examples described later, and are preferred anti-SARS-CoV-2 antibodies.

The form of the antibody of the present invention is not limited as long as it has at least one of the above structural domains. The antibody of the present invention may be a single-domain antibody (also referred to as a nanobody) such as a VHH antibody, or a single-chain antibody, a heavy-chain antibody, a multimer of variable region fragments ligated via a peptide linker or the like, e.g., a dimer, or a multimer of a variable region fragment ligated with one or more variable region fragments with different antigen specificity.

The antibody of the present invention may be humanized. The humanized antibody can be administered to a human, and thus can be applied as a medicament.

The method for producing the antibody of the present invention is not particularly limited, and the antibody of the present invention can be easily produced by techniques known in this technical field. For example, the antibody of the present invention can be produced by a combination of peptide solid-phase synthesis and native chemical ligation (NCL), or by genetic engineering. In a preferred method, a nucleic acid encoding the antibody of the present invention is incorporated into a suitable vector, and the vector is introduced into host cells to produce a recombinant antibody.

Examples of host cells used in the production of the recombinant antibody include *Escherichia coli, Bacillus subtilis,* fungi, animal cells, plant cells, baculovirus/insect cells, yeast cells, and the like. Vectors suitable for various host cells can be used as expression vectors for antibody expression. Examples of expression vectors which can be used include *Escherichia* coli-derived vectors, such as pBR322, pBR325, pUC12, and pUC13; *Bacillus* subtilis-derived vectors, such as pUB110, pTP5, and pC194; shuttle vectors which can be shared between *Escherichia coli* and *Bacillus subtilis,* such as pHY300PLK; yeast-derived vectors, such as pSH19 and pSH15; bacteriophages, such as λ phage; viruses, such as adenovirus, adeno-associated virus, lentivirus, vaccinia virus, and baculovirus; and vectors obtained by modifying these.

These expression vectors have a replication origin, a selection marker, and a promoter suitable for each vector, and may have, if necessary, an enhancer, a transcription termination sequence (terminator), a ribosome-binding site, a polyadenylation signal, and the like. Further, in order to facilitate the purification of the expressed polypeptide, a base sequence for expression may be inserted into the expression vector by fusing FLAG tag, His tag, HA tag, GST tag, and the like.

When the expressed antibody of the present invention is extracted from the cultured bacteria or cultured cells, the bacterial or cultured cells are collected by a known method after culture, and then suspended in an appropriate buffer, and the bacterial or cells are broken by ultrasonication, lysozyme, and/or freeze-thaw and the like, followed by centrifugation and filtration to obtain a soluble extract. The target antibody can be obtained from the obtained extract by appropriately combining known separation and purification methods.

Known separation and purification methods which can be used include methods based on solubility, such as salting out and solvent precipitation; methods mainly based on differences in molecular weight, such as dialysis, ultrafiltration, gel filtration, and SDS-PAGE; methods based on charge differences, such as ion-exchange chromatography; methods based on specific affinity, such as affinity chromatography; methods based on differences in hydrophobicity, such as reversed-phase high-performance liquid chromatography; methods based on differences in isoelectric points, such as isoelectric focusing; and the like.

Since the antibody of the present invention binds to the N-protein of SARS-CoV-2, it is possible to confirm the presence or absence of SARS-CoV-2 in a sample which contains or may contain SARS-CoV-2 by contacting this antibody with the test sample.

Specifically, detection of SARS-CoV-2 using the antibody of the present invention includes a step of contacting the antibody of the present invention with a test sample to form a complex of the antibody of the present invention and SARS-CoV-2 in the test sample, and a step of detecting SARS-CoV-2 in the complex.

In the detection of virus-specific antibodies in serum, a part of an antigen containing the N-protein of SARS-CoV-2 is added to bind to an anti-SARS-CoV-2 antibody (e.g., serum antibody) contained in an immobilized test sample (serum), and the antibody of the present invention can be used as an antibody which can confirm the presence of the SARS-CoV-2 antigen in the bound state.

Examples of test samples include biological samples, such as tracheal swab, nasal swab, pharyngeal swab, nasal lavage fluid, nasal aspirate, nasal discharge nasal mucus, saliva, sputum, blood, serum, urine, feces, tissues, cells, and tissue or cell debris; as well as samples collected from solid surfaces which may have viruses on them, such as doorknobs and toilet bowls. In terms of binding to the N-protein, it is preferable that the virus in the test sample is dissolved in a solution containing a surfactant and the like, and is allowed to bind to the antibody in the solution. The antibody may be immobilized on a solid phase or may not be immobilized on a solid phase.

The step of detecting SARS-CoV-2 in the above complex can be performed, for example, by allowing the complex to react with an anti-SARS-CoV-2 antibody which recognizes the epitope of the complex different from that of the antibody of the present invention. Alternatively, SARS-CoV-2 in the complex may be detected in a liquid phase by homogeneous assay.

The antibody of the present invention can also be used as a constituent of a kit for detecting SARS-CoV-2. The kit can be used as a diagnostic agent for SARS-CoV-2 infection (COVID-19) or as a tool for the development of therapeutic agents for SARS-CoV-2 infection.

In addition to the anti-SARS-CoV-2 antibody of the present invention, the detection kit may contain reagents and equipment required for detection, such as an antibody which recognizes the antibody of the present invention, or N-protein antibody which recognizes an epitope different from that of the antibody of the present invention, a solid-phase carrier, a buffer, an enzyme reaction stop solution, a microplate reader, and the like.

In the detection kit, the antibody of the present invention may be immobilized on a solid phase. Examples of the solid phase include beads, membranes, side and bottom surfaces of reaction vessels, plate substrates such as glass slides, well substrates such as immunoplates, and the like. The antibody of the present invention is directly or indirectly immobilized thereon.

The antibody of the present invention is expected to bind to the N-protein of SARS-CoV-2 to inhibit viral replication within cells. Therefore, the antibody of the present invention can also be administered to mammals and used as a medicament for preventing or treating SARS-CoV-2 infection. Examples of mammals include humans, mice, rats, hamsters, guinea pigs, rabbits, cats, dogs, monkeys, cows, horses, pigs, and the like; humans are preferred.

When the antibody of the present invention is used as a medicament, the dosage form thereof may be oral or parenteral, and such a medicament can be used in combination with well-known pharmaceutically acceptable non-toxic carriers and diluents, as appropriate. Parenteral administration typically includes injections; however, administration by inhalation together with sprays and the like is also possible.

In the medicament, the content of the antibody of the present invention in the composition can be suitably adjusted.

The medicament can be applied in such a manner that an effective amount of the antibody of the present invention is administered to a patient at intervals of from about one to several times a week. Preferred administration methods in this case include intravenous injection, infusion, and the like.

Regarding the embodiments describe above, the present invention further discloses the following aspects.

<1> An antibody that binds to SARS-CoV-2, having one or more structural domains comprising CDRs shown in the following (a) or (b):

(a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or

(b) CDR1 consisting of the amino acid sequence set forth in SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

<2> The antibody according to <1>, which binds to N-protein.

<3> The antibody according to <1> or <2>, which is a VHH antibody, a heavy-chain antibody, or a VHH antibody multimer.

<4> The antibody according to <3>, wherein the VHH antibody multimer is a multimer in which the structural domains are linked to each other.

<5> The antibody according to <3>, wherein the VHH antibody multimer is a dimer in which two of the structural domains are linked to each other.

<6> The antibody according to <3>, wherein the VHH antibody multimer is a multimer in which one or more of the structural domains are linked to one or more structural domains with different antigen specificity from the former structural domains.

<7> The antibody according to any one of <1> to <6>, wherein the substitution of one amino acid with another amino acid in the amino acid sequence of SEQ ID NO: 1 is substitution of glycine residue at position 1 with an arginine residue, substitution of phenylalanine residue at position 2 with a leucine residue, substitution of phenylalanine residue at position 4 with a leucine residue, substitution of lysine residue at position 6 with an asparagine residue, substitution of phenylalanine at position 7 residue with a leucine residue or a cysteine residue, substitution of tryptophan residue ay position 8 with a cysteine residue, or substitution of methionine residue at position 9 with a valine residue or an arginine residue.

<8> The antibody according to any one of <1> to <6>, wherein the substitution of one amino acid with another amino acid in the amino acid sequence of SEQ ID NO: 2 is substitution of phenylalanine residue at position 1 with a leucine residue or substitution of asparagine residue at position 10 with an aspartic acid residue.

<9> The antibody according to any one of <1> to <6>, wherein the substitution of one amino acid with another amino acid in the amino acid sequence of SEQ ID NO: 3 is substitution of glycine residue at position 1 with a cysteine residue, substitution of threonine residue at position 6 with an alanine residue or a proline residue, substitution of valine residue at position 9 with an isoleucine residue, or substitution of histidine residue at position 12 with an asparagine residue or an aspartic acid residue.

<10> The antibody according to any one of <1> to <9>, wherein the structural domain has a first framework region (FR1), CDR1, a second framework region (FR2), CDR2, a third framework region (FR3), CDR3, and a fourth framework region (FR4) in this order.

<11> The antibody according to <10>, wherein FR1 to FR4 consist of the following amino acid sequences:

FR1: the amino acid sequence of SEQ ID NO: 7 or 8, or an amino acid sequence having 80% or more identity to the amino acid sequence
FR2: the amino acid sequence of SEQ ID NO: 9 or 10, or an amino acid sequence having 80% or more identity to the amino acid sequence
FR3: the amino acid sequence of SEQ ID NO: 11 or 12, or an amino acid sequence having 80% or more identity to the amino acid sequence
FR4: the amino acid sequence of SEQ ID NO: 13, or an amino acid sequence having 80% or more identity to the amino acid sequence

<12> The antibody according to <10>, which has a structural domain in which FR1, CDR1, FR2, CDR2, FR3, CDR3, and FR4 are linked in this order, and in which CDR1 consists of the amino acid sequence of SEQ ID NO: 1, CDR2 consists of the amino acid sequence of SEQ ID NO: 2, CDR3 consists of the amino acid sequence of SEQ ID NO: 3, FR1 consists of the amino acid sequence of SEQ ID NO: 7, FR2 consists of the amino acid sequence of SEQ ID NO: 9, FR3 consists of the amino acid sequence of SEQ ID NO: 11, and FR4 consists of the amino acid sequence of SEQ ID NO: 13.

<13> The antibody according to <10>, which has a structural domain in which FR1, CDR1, FR2, CDR2, FR3, CDR3, and FR4 are linked in this order, and in which CDR1 consists of the amino acid sequence of SEQ ID NO: 4, CDR2 consists of the amino acid sequence of SEQ ID NO: 5, CDR3 consists of the amino acid sequence of SEQ ID NO: 6, FR1 consists of the amino acid sequence of SEQ ID NO: 8, FR2 consists of the amino acid sequence of SEQ ID NO: 10, FR3 consists of the amino acid sequence of SEQ ID NO: 12, and FR4 consists of the amino acid sequence SEQ ID NO: 13.

<14> A nucleic acid encoding the antibody according to <1>.

<15> A method for detecting SARS-CoV-2 in a sample, comprising a step of contacting an antibody that binds to SARS-CoV-2 with a test sample, the antibody having one or more structural domains comprising CDRs shown in the following (a) or (b):

(a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or

(b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

<16> A kit for detecting SARS-CoV-2, comprising the antibody according to any one of <1> to <13>.

<17> A medicament comprising the antibody according to any one of <1> to <13>.

<18> The medicament according to <17>, for use in prevention or treatment of SARS-CoV-2 infection.

<19> Use of the antibody according to any one of <1> to <13>, for producing a medicament.

<20> The antibody according to any one of <1> to <13>, for use as a medicament.

<21> The use according to <19> or the antibody according to <20>, wherein the medicament is a medicament for preventing or treating SARS-CoV-2 infection.

<22> A method for preventing or treating SARS-CoV-2 infection, comprising administering the antibody according to any one of <1> to <13> to a subject in need thereof.

Examples

Example 1: Screening of anti-SARS-CoV-2 N-protein antibody

<Materials and methods>

1. Target molecules to be screened

As the target molecules, SARS-CoV-2 Nucleoprotein, His-Tag (*E. coli*) (His-tagged N-protein, The Native Antigen Company) was used.

2. Synthesis of cDNA display

(1) Synthesis of early DNA

4,500 ng of AlipLib L hinge 2nd PCR product and 4,500 ng of AlipLib S hinge 2nd PCR product (Epsilon Molecular Engineering), which are DNAs for synthesizing a VHH-encoding cDNA display, were amplified by PCR. The reaction solution was prepared by mixing, for each sample, 25 μL of KAPA HiFi HotStart Ready Mix (2X) (Kapa Biosystems), 1.5 μL of 10 μM forward primer (5'-GATCCCGCGAAATTAATACGACTCACTATAGGG-3' (SEQ ID NO: 16)), 1.5 μL of 10 μM reverse index primer (5'-TTTCCACGCCGCCCCCCGTCCT-3' (SEQ ID NO: 17)), and 300 ng of DNA, and adjusting the volume to 50 μL with nuclease-free water. PCR was performed at 95°C for 5 minutes, followed by 5 cycles of 98°C for 20 seconds, 65°C for 15 seconds, and 72°C for 30 seconds, and then 72°C for 5 minutes. Agencourt AMPure XP (Beckman Coulter) was used to purify the PCR product. 1.8 μL of beads were added per μL of the PCR product, and the mixture was mixed by pipetting and then allowed to stand for 5 minutes. After the resultant was allowed to stand on a magnetic plate until clear, the supernatant was removed. 1.4 mL of 70% ethanol was added, the mixture was allowed to stand for 30 seconds, and the supernatant was then removed. This ethanol washing was performed three times. After the supernatant was removed, the resultant was allowed to stand on the magnetic plate for 3 minutes, and the beads were air-dried. The tube was removed from the magnetic plate, 100 μL of nuclease-free water was added, and the beads were then suspended by pipetting and allowed to stand for 5 minutes. After the tube was allowed to stand on the magnetic plate for 2 minutes, the supernatant was collected. After purification, DNA was quantified by NanoPad DS-11FX (DeNovix), and the PCR products derived from AlipLib L hinge 2nd PCR product and 4,500 ng of AlipLib S hinge 2nd PCR product were mixed in equal amounts (this mixture is called early DNA).

(2) In vitro transcription

The T7 RiboMAX Express Large Scale RNA Production System (Promega) was used. 25 μL of RiboMAX (trademark) Express T7 2X Buffer, 20 pmol of the PCR product (early DNA or PCR product after each selection), and 5 μL of Enzyme Mix were mixed, and the mixture was incubated at 37°C for 30 minutes. Subsequently, 5 μL of RQ1 RNase-Free DNase was added, followed by incubation at 37°C for 15 minutes. RNAClean XP (Beckman Coulter) was used to purify the transcript. 1.8 μL of beads were added per μL of the transcript, and the mixture was mixed by pipetting and then allowed to stand for 5 minutes. After the resultant was allowed to stand on a magnetic plate until clear, the supernatant was removed. 200 μL of 70% ethanol was added, the mixture was allowed to stand for 30 seconds, and the supernatant was then removed. This ethanol washing was performed three times. After the supernatant was removed, the resultant was allowed to stand on the magnetic plate for 10 minutes, and the beads were air-dried. The tube was removed from the magnetic plate, 20 μL of nuclease-free water was added, and the beads were then suspended by pipetting and allowed to stand for 5 minutes. After the tube was allowed to stand on the magnetic plate for 1 minute, the supernatant was collected. After purification, RNA was quantified by NanoPad DS-11FX (DeNovix).

(3) Ligation of mRNA and puromycin linker

The reaction solution was prepared by mixing 4 μL of 0.25M Tris-HCl (pH: 7.5), 4 μL of 1M NaCl, 20 pmol of mRNA, and 20 pmol of cnvK riboG linker, and adjusting the volume to 20 μL with nuclease-free water. Annealing was performed using the ProFlex PCR System (Life Technologies) under the incubation conditions of 90°C for 2 minutes, 70°C for 1 minute, 25°C for 30 seconds, and 4°C. The ramp rate was set to 0.1°C/sec. Subsequently, Handheld UV Lamp, 6W, UVGL-58, 254/365 nm, 100V (Analytik Jena US, An Endress+Hauser Company) was used to irradiate ultraviolet rays with a wavelength of 365 nm for 5 minutes. The mRNA-linker conjugate was protected from light and cooled on ice until use.

(4) Cell-free translation

For the synthesis of an mRNA-VHH conjugate from the mRNA-linker conjugate, the Rabbit Reticulocyte Lysate System, Nuclease Treated (Promega) was used. 10.5 μL of nuclease-free water, 1.5 μL of 20x Translation Mix, 52.5 μL of rabbit reticulocyte lysate, 1.5 μL of RNasin (trademark) Ribonuclease Inhibitor (40 U/μL) (Promega), and 9 μL of the mRNA-linker conjugate were mixed. After this reaction solution was incubated at 37°C for 15 minutes, 36 μL of IVV formation buffer (3M KCl, 1M MgCl$_2$) mixture was added, and the resulting mixture was further reacted at 37°C for 20 minutes, thereby synthesizing an mRNA-VHH conjugate.

(5) Immobilization on streptavidin magnetic beads

60 μL of 2x binding buffer (20 mM Tris-HCl, 2M NaCl, 0.2% (w/v) Tween 20, and 2 mM EDTA, pH: 8) was added to 60 μL of Dynabeads My One Streptavidin C1 (Thermo Fisher Scientific), and the mixture was suspended by pipetting for 1 minute. The resultant was allowed to stand on a magnetic plate for 1 minute, and the supernatant was discarded. This washing was performed twice. 75 μL of the mRNA-VHH conjugate, 75 μL of 2x binding buffer, and the washed streptavidin magnetic beads were mixed and incubated at room temperature for 30 minutes. After the resultant was allowed to stand on the magnetic plate for 1 minute, the supernatant was removed. 200 μL of binding buffer (10 mM Tris-HCl, 1M NaCl, 0.1% (w/v) Tween 20, and 1 mM EDTA, pH: 8) was added thereto, followed by pipetting for 1 minute, and then removing the supernatant. This washing was performed twice.

(6) Reverse transcription reaction

55.5 μL of nuclease-free water, 15 μE of 5x ReverTra Ace Buffer (Toyobo Life Science), 3 μL of 25 mM dNTP Mix, and 1.5 μL of ReverTra Ace (100 U/μL) (Toyobo Life Science) were mixed. The streptavidin magnetic beads on which the mRNA-VHH conjugate was immobilized were added to this reaction solution, and the mixture was incubated at 42°C for 30 minutes to perform a reverse transcription reaction, thereby synthesizing a cDNA-VHH conjugate.

(7) Cutting out from beads

His-tag wash buffer (20 mM sodium phosphate, 500 mM NaCl, 5 mM imidazole, and 0.05% (w/v) Tween 20, pH: 7.4) was added to the cDNA-VHH conjugate immobilized on the streptavidin magnetic beads, and the mixture was suspended by pipetting for 1 minute. The resultant was allowed to stand on a magnetic plate for 1 minute, and the supernatant was discarded. Subsequently, 30 μL of 10U RNase T1 (Thermo Fisher Scientific)-containing His-tag wash buffer was added, and the mixture was suspended by pipetting for 1 minute and then allowed to stand at 37°C for 15 minutes, thereby eluting the cDNA-VHH conjugate.

(8) Purification

30 μL of His Mag Sepharose Ni Beads (GE Health Care) were allowed to stand on a magnetic plate for 1 minute, the supernatant was discarded, and the resultant was then resuspended with His-tag wash buffer. This washing operation was performed twice. The eluate of the cDNA-peptide conjugate and the suspension of the His Mag Sepharose Ni Beads were mixed, incubated at room temperature for 30 minutes, and then allowed to stand on a magnetic plate for 1 minute, and the supernatant was discarded. 200 μL of His-tag wash buffer was added thereto, and the mixture was suspended by pipetting for 1 minute. The resultant was allowed to stand on the magnetic plate for 1 minute, and the supernatant was discarded. This washing operation was performed twice. 10 μL of His-tag elution buffer (20 mM sodium phosphate, 500 mM NaCl, 250 mM imidazole, and 0.05% (w/v) Tween 20, pH: 7.4) was added thereto, and the mixture was incubated at room temperature for 15 minutes, thereby eluting the purified cDNA-VHH conjugate (referred to as the cDNA display).

3. Selection

(1) Immobilization of target molecules and blocking

100 μL of His-tagged N-protein adjusted with PBS to 50 μg/mL (1st round), 10 μg/mL (2nd round), 5 μg/mL (3rd round), and 1 μg/mL (4th and 5th rounds) was added to each well of Nunc-Immuno (trademark) Plate II (Thermo Fisher Scientific), and after sealing, the resultant was allowed to stand at 4°C overnight. After using a pipette to carefully remove the His-tagged N protein and PBS which were not adsorbed to the wells, 200 μL of 5% (w/v) skim milk/PBST (0.05% (w/v) Tween 20-containing PBS) was added, and the mixture was incubated at room temperature for 1 hour. After using a pipette to carefully remove the 5% (w/v) skim milk/PBST (0.05% (w/v) Tween 20-containing PBS), 200 μL of HBST (20 mM HEPES, 500 mM NaCl, and 0.02% (w/v) Tween 20, pH: 7.2) was added thereto, and the mixture was allowed to stand for 5 minutes, followed by careful removal using a pipette. This washing operation was performed three times.

(2) Pre-incubation (performed after the 2nd round selection)

200 μL of 5% (w/v) skim milk/PBST (0.05% (w/v) Tween 20-containing PBS) was added to each well of Nunc-Immuno (trademark) Plate II (Thermo Fisher Scientific), and after sealing, the resultant was allowed to stand at 4°C overnight. After using a pipette to carefully remove the skim milk which was not adsorbed to the wells, 200 μL of HBST (20 mM HEPES, 500 mM NaCl, and 0.02% (w/v) Tween 20, pH: 7.2) was added thereto, and the mixture was allowed to stand for 5 minutes, followed by careful removal using a pipette. This washing operation was performed three times.

The cDNA display was adjusted to 200 pmol for the 1st round selection, 12 pmol for the 2nd round selection, and 6 pmol for the 3rd round and subsequent selections. Each cDNA display was prepared to 100 μL by adding HBST (20 mM HEPES, 500 mM NaCl, and 0.02% (w/v) Tween 20, pH: 7.2). Of these, each cDNA display after the 2nd round was added to wells blocked with skim milk, followed by incubation at room temperature for 1 hour. Thereafter, all the supernatants were collected. As a result, cDNA-VHH conjugates that binds to the blocking agent were removed.

(3) Selection

The cDNA display (after the 2nd round, the preincubated cDNA display) was added to wells to which the N-protein was immobilized, followed by incubation at room temperature for 1 hour. After using a pipette to carefully remove the cDNA display solution, 200 µL of HBST was added thereto, and the mixture was incubated at room temperature for 5 minutes, followed by careful removal using a pipette. This washing operation was performed 10 times. 100 µL of 100 mM tris(hydroxymethyl)aminomethane (pH: 11) was added thereto, and after careful pipetting, the resultant was allowed to stand at room temperature for 5 minutes. After standing, the supernatant was collected in a new tube.

4. PCR amplification

The eluate was subjected to PCR. The reaction solution was prepared by mixing, for each sample, 25 µL of KAPA HiFi HotStart Ready Mix (2X) (Kapa Biosystems), 1.5 µL of 10 µM forward primer, 1.5 µL of 10 µM reverse index primer, 12.5 µL of the cDNA display after selection, and 9.5 µL of nuclease-free water. The primers used were 5'-GATCCCGCGAAATTAATACGACTCACTATAGGGGAAGTATTTTTACAACAATTACCA ACA-3' (SEQ ID NO: 18) and 5'-TTTCCACGCCGCCCCCCGTCCT-3' (SEQ ID NO: 17). PCR was performed at 95°C for 2 minutes, followed by 22 cycles (1st round) or 24 cycles (2nd round and subsequent rounds) of 98°C for 20 seconds, 68°C for 15 seconds, and 72°C for 20 seconds, and then 72°C for 5 minutes. Agencourt AMPure XP (Beckman Coulter) was used to purify the PCR product. 1.8 µL of beads were added per µL of the PCR product, and the mixture was mixed by pipetting and then allowed to stand for 5 minutes. After the resultant was allowed to stand on a magnetic plate until clear, the supernatant was removed. 200 µL of 70% ethanol was added, the mixture was allowed to stand for 30 seconds, and the supernatant was then removed. This ethanol washing was performed twice. After the supernatant was removed, the resultant was allowed to stand on the magnetic plate for 5 minutes, and the beads were air-dried. The tube was removed from the magnetic plate, 45 µL of nuclease-free water was added thereto, and the beads were then suspended by pipetting and allowed to stand for 5 minutes. After the tube was allowed to stand on the magnetic plate for 1 minute, the supernatant was collected. The PCR product obtained herein was used as a template, and a cDNA display was synthesized for use in the next round of selection.

5. Extraction of VHH antibody candidate sequences

(1) Quantification of PCR product and concentration adjustment

The PCR products were quantified using the PicoGreen (trademark) dsDNA reagent kit (Thermo Fisher Scientific). In accordance with the quantitative value, each sample was prepared to a concentration of 50 ng/mL.

(2) Sequence preparation

1st PCR was performed. The reaction solution was prepared by mixing, for each sample, 12.5 µL of KAPA HiFi HotStart Ready Mix (2X) (Kapa Biosystems), 0.5 µL of 10 µM forward primer, 0.5 µL of 10 µM reverse primer, 2.5 µL of the PCR product described in the above paragraph, and 9 µL of nuclease-free water. The primers used were 5'-TCGTCGGCAGCGTCAGATGTGTATAAGAGACAGNNNNATGGCTGAGGTGCAGCTCGT G-3' (SEQ ID NO: 19) and 5'-GTCTCGTGGGCTCGGAGATGTGTATAAGAGACAGNNNNTGATGATGATGGCTACCAC CTCCCG-3' (SEQ ID NO: 20). PCR was performed at 95°C for 3 minutes, followed by 16 cycles of 98°C for 20 seconds, 62°C for 15 seconds, and 72°C for 20 seconds, and then 72°C for 5 minutes.
Agencourt AMPure XP (Beckman Coulter) was used to purify the PCR product. 1.8 µL of beads were added per µL of the PCR product, and the mixture was mixed by pipetting and then allowed to stand for 5 minutes. After the resultant was allowed to stand on a magnetic plate until clear, the supernatant was removed. 200 µL of 70% ethanol was added, the mixture was allowed to stand for 30 seconds, and the supernatant was then removed. This ethanol washing was performed twice. After the supernatant was removed, the resultant was allowed to stand on the magnetic plate for 5 minutes, and the beads were air-dried. The tube was removed from the magnetic plate, 20 µL of nuclease-free water was added thereto, and the beads were then suspended by pipetting and allowed to stand for 5 minutes. After the tube was allowed to stand on the magnetic plate for 1 minute, the supernatant was collected.
Subsequently, index PCR was performed. The reaction solution was prepared by mixing, for each sample, 12.5 µL of KAPA HiFi HotStart Ready Mix (2X) (Kapa Biosystems), 1 µL of 10 µM forward index primer (Nextera XT Index Kit v2, Illumina), 1 µL of 10 µM reverse index primer (Nextera XT Index Kit v2, Illumina), 2.5 µL of template DNA, and 8 µL of nuclease-free water. PCR was performed at 95°C for 3 minutes, followed by 8 cycles of 98°C for 20 seconds, 55°C for 15 seconds, and 72°C for 30 seconds, and then 72°C for 5 minutes. Agencourt AMPure XP (Beckman Coulter) was used to purify the PCR product. After the size of the PCR product was confirmed by polyacrylamide gel electrophoresis, the PCR product was quantified using the Quant-iT PicoGreen dsDNA Assay Kit (Thermo Fisher Scientific), and the molar concentration was calculated in accordance with the following equation (1). Based on the obtained quantitative value, the concentration was adjusted to 4 nM by dilution with nuclease-free water (the resultant is called the sample).

$$\text{DNA concentration [ng/µL]}/(660 \text{ [g/mol]} \times 550 \text{ [bp]}) \times 10^6 = \text{DNA concentration [nM]} \dots (1)$$

(3) Sequencing

Preparation was performed in accordance with the recommended protocol when using 4 nM. The final concentration of the denatured and diluted sample was adjusted to 7 pM. The PhiX Control in the sample was adjusted to 5%. MiSeq (Illumina) and MiSeq Reagent Nano Kit v2 500 cycle (Illumina) were used to perform sequencing.

(4) Extraction of amino acid sequence of VHH antibody from sequence data and measurement of the number of duplicates

The sequence data demultiplexed by the MiSeq Controller was analyzed. Base sequence regions encoded by VHH antibodies were extracted from the sequence data and translated into amino acid sequences. Thereafter, the number of amino acid sequences which completely matched each amino acid sequence was counted.

(5) Selection of anti-SARS-CoV-2 VHH antibody candidate sequences

Based on the number of clones for each amino acid sequence of VHH antibody commonly appearing after the 4th and 5th round selections, the appearance frequency in each VHH antibody was calculated. As a result, CoVHH-N1 (SEQ ID NO: 14) and CoVHH-N2 (SEQ ID NO: 15) frequently appeared, and were selected as antibodies with high affinity for the N protein (Table 1).

[Table 1]

| VHH name | Appearance frequency (%) | |
| --- | --- | --- |
| | 4th round | 5th round |
| CoVHH-N1 | 0.77 | 3.31 |
| CoVHH-N2 | 0.05 | 0.20 |

In the amino acid sequence of CoVHH-N1, positions 1 to 26 represent FR1 (SEQ ID NO: 7), positions 27 to 36 represent CDR1 (SEQ ID NO: 1), positions 37 to 50 represent FR2 (SEQ ID NO: 9), positions 51 to 60 represent CDR2 (SEQ ID NO: 2), positions 61 to 99 represent FR3 (SEQ ID NO: 11), positions 100 to 112 represent CDR3 (SEQ ID NO: 3), and positions 113 to 123 represent FR4 (SEQ ID NO: 13).

In the amino acid sequence of CoVHH-N2, positions 1 to 26 represent FR1 (SEQ ID NO: 8), positions 27 to 36 represent CDR1 (SEQ ID NO: 4), positions 37 to 50 represent FR2 (SEQ ID NO: 10), positions 51 to 60 represent CDR2 (SEQ ID NO: 5), positions 61 to 99 represent FR3 (SEQ ID NO: 12), 100 to 108 represent CDR3 (SEQ ID NO: 6), and positions 109 to 119 represent FR4 (SEQ ID NO: 13).

(6) Extraction of CDR region amino acid sequence of VHH antibody

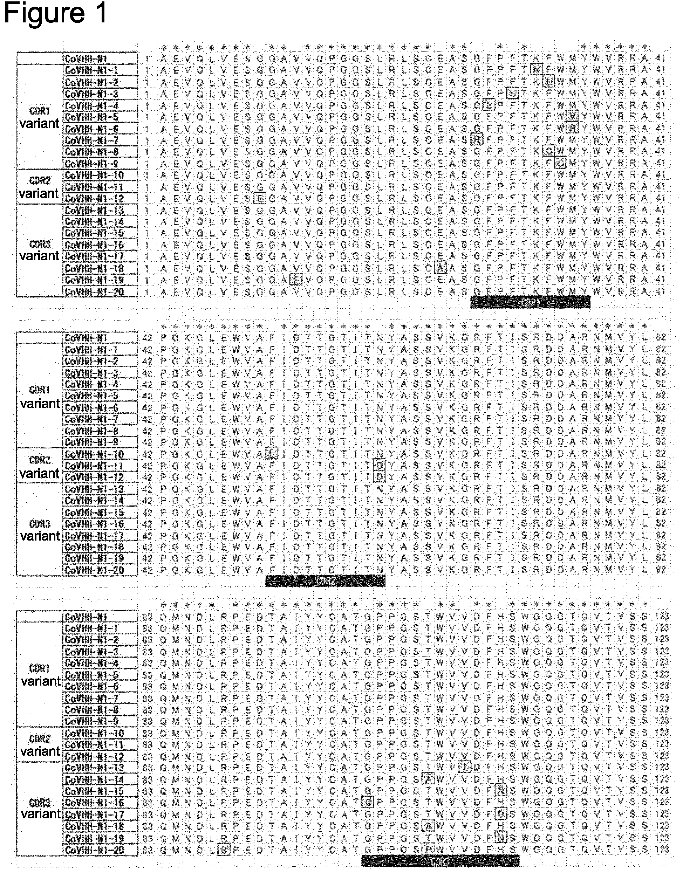

VHHs commonly appearing after the 4th and 5th round selections were compared with the amino acid sequence of CoVHH-N1, and CoVHH-N1 variants (called CDR1 variants, CDR2 variants, or CDR3 variants) in which one amino acid contained in each CDR region of CoVHH-N1 was substituted with another amino acid were selected (Figure 1). The CDR1 variants of CoVHH-N1 having substitution of one amino acid in CDR1 are CoVHH-N1-1 (SEQ ID NO: 21), CoVHH-N1-2 (SEQ ID NO: 22), CoVHH-N1-3 (SEQ ID NO: 23), CoVHH-N1-4 (SEQ ID NO: 24), CoVHH-N1-5 (SEQ ID NO: 25), CoVHH-N1-6 (SEQ ID NO: 26), CoVHH-N1-7 (SEQ ID NO: 27), CoVHH-N1-8 (SEQ ID NO: 28), and CoVHH-N1-9 (SEQ ID NO: 29).

The CDR1 variants of CoVHH-N1 having substitution of one amino acid in CDR2 are CoVHH-N1-10 (SEQ ID NO: 30), CoVHH-N1-11 (SEQ ID NO: 31), and CoVHH-N1-12 (SEQ ID NO: 32). The CDR1 variants of CoVHH-N1 having substitution of one amino acid in CDR3 are CoVHH-N1-13 (SEQ ID NO: 33), CoVHH-N1-14 (SEQ ID NO: 34), CoVHH-N1-15 (SEQ ID NO: 35), CoVHH-N1-16 (SEQ ID NO: 36), CoVHH-N1-17 (SEQ ID NO: 37), CoVHH-N1-18 (SEQ ID NO: 38), CoVHH-N1-19 (SEQ ID NO: 39), and CoVHH-N1-20 (SEQ ID NO: 40).

Example 2: VHH production by protease-deficient recombinant *Bacillus subtilis*

(1) Artificial synthesis of genes

For the VHH amino acid sequence to be synthesized, a His-tagged sequence (SEQ ID NO: 41, GGGSHHHHHH) was added via a linker sequence to the C-terminal side of CoVHH-N1 (SEQ ID NO: 14) or CoVHH-N2 (SEQ ID NO: 15). The thus-synthesized VHHs are called His-tagged CoVHH-N1 (SEQ ID NO: 42) and His-tagged CoVHH-N2 (SEQ ID NO: 43). As the artificially synthesized genes for synthesizing His-tagged CoVHH-N1 and His-tagged CoVHH-N2, an artificially synthesized gene for His-tagged CoVHH-N1 (SEQ ID NO: 44) and an artificially synthesized gene for CoVHH-N2 (SEQ ID NO: 45) were artificially synthesized by Eurofins and used for experiments.

(2) Construction of VHH expression plasmid

Using recombinant plasmid pHY-S237 (JP-A-2014-158430) as a template, the plasmid sequence was amplified by PCR using a primer set of 5'-GATCCCCGGGAATTCCTGTTATAAAAAAAGG-3' (SEQ ID NO: 46) and 5'-ATGATGTTAAGAAAGAAAACAAAGCAG-3' (SEQ ID NO: 47), and PrimeSTAR Max DNA Polymerase (Takara). Using the 168 strain genome as a template, spoVG gene-derived promoter DNA was amplified by PCR using a primer set of 5'-GAATTCCCGGGGGATCTAAGAAAAGTGATTCTGGGAGAG-3' (SEQ ID NO: 48) and 5'-CTTTCTTAACATCATAGTAGTTCACCACCTTTTCCC-3' (SEQ ID NO: 49). The obtained promoter DNA was incorporated into the plasmid sequence using the In-Fusion HD Cloning Kit (Takara), and a VHH expression plasmid ligated with spoVG promoter was constructed. The plasmid sequence was amplified by PCR using a primer set of 5'-TGCTGCAAGAGCTGCCGGAAATAAA-3' (SEQ ID NO: 50) and 5'-TCTATTAAACTAGTTATAGGG-3' (SEQ ID NO: 51), and PrimeSTAR Max DNA Polymerase (Takara). DNA containing the artificially synthesized gene of (1) was incorporated into the obtained PCR fragments using the In-Fusion HD Cloning Kit (Takara), and VHH expression plasmids containing each of the artificially synthesized VHH genes were constructed.

(3) Production of recombinant *Bacillus subtilis*

A strain with deletion of extracellular protease genes (epr, wprA, mpr, nprB, bpr, nprE, vpr, and aprE) was produced from *Bacillus subtilis* 168 strain (hereinafter referred to as 168 strain) in accordance with the method described in JP-A-2006-174707. Further, in accordance with the method described in JP-B-4336082, the sigF gene involved in sporulation was deleted from *Bacillus subtilis* strain Dpr8 with deletion of all of the above eight extracellular protease genes. The obtained multiple-extracellular protease-deficient strain is referred to as Dpr8ΔsigF.

Plasmid introduction into the Dpr8ΔsigF strain was performed by the protoplast method shown below. Glycerol-stocked *Bacillus subtilis* was inoculated in 1 mL of LB liquid medium and cultured with shaking at 30°C and 210 rpm overnight. On the next day, 10 μL of this culture solution was inoculated in 1 mL of fresh LB liquid medium and cultured with shaking at 37°C and 210 rpm for about 2 hours. This culture solution was collected in a 1.5-mL tube and centrifuged at 12,000 rpm for 5 minutes. After removing the supernatant, the resulting pellet was suspended in 500 μL of SMMP containing 4 mg/mL of Lysozyme (SIGMA), followed by incubation at 37°C for 1 hour. After centrifugation at 3,500 rpm for 10 minutes, the supernatant was removed, and the resulting pellet was suspended in 400 μL of SMMP. 33 μL of this suspension was mixed with various plasmids, 100 μL of 40% PEG was further added thereto, and the mixture was vortexed. 350 μL of SMMP was added to this solution and mixed by inverting. After shaking at 30°C and 210 rpm for 1 hour, the entire amount was applied to DM3 agar plate, followed by incubation at 30°C for 2 to 3 days.

(4) VHH production

The recombinant *Bacillus subtilis* produced in (3) was inoculated in LB medium containing 1 mL of 50 ppm tetracycline, reciprocally shaken at 30°C overnight, and used as a pre-culture solution. Regarding Dpr8ΔsigF, 1% of the pre-culture solution was inoculated in 20 mL of 2xL-Mal medium placed in an Erlenmeyer flask with baffles, and cultured by shaking at 30°C for 72 hours. At the end of culture, 1 mL of the culture solution was centrifuged in a microtube at 4°C and 15,000 rpm for 5 minutes, and the supernatant was collected. Each VHH was purified using Ni-NTA agarose beads (FUJIFILM Wako Pure Chemical Corporation) in accordance with the protocol of the kit. The purified protein was dissolved in PBS (30 mM imidazole).

(5) Confirmation by SDS-PAGE

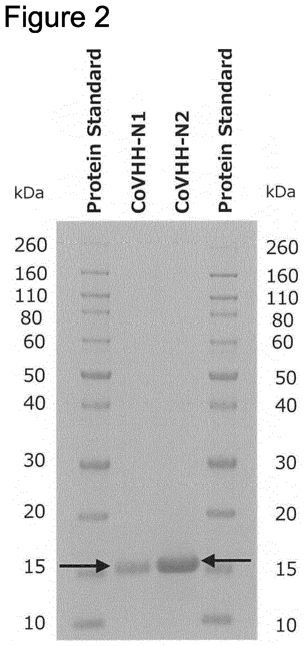

An aqueous solution of 6.5 µL of His-tagged CoVHH-N1 or His-tagged CoVHH-N2 (both 200 µg/mL), 2.5 µL of NuPAGE (trademark) LDS Sample Buffer (4X) (Thermo Fisher Scientific), and 1 µL of NuPAGE (trademark) Sample Reducing Agent (10X) (Thermo Fisher Scientific) were mixed and heated at 100°C. Thereafter, 10 µL of the sample solution was applied to gel wells. As a molecular weight marker, 5 µL of Novex (trademark) Sharp Pre-stained Protein Standard (Thermo Fisher Scientific) was also applied to the gel wells. The gel used was NuPAGE (trademark) 10%, Bis-Tris, 1.0 mm, Mini Protein Gel, 12-well (Thermo Fisher Scientific). The electrophoresis buffer used was prepared by 20-fold diluting NuPAGE (trademark) MES SDS Running Buffer (20X) (Thermo Fisher Scientific) with water. XCell SureLock (trademark) Mini-Cell Electrophoresis System (Thermo Fisher Scientific) was used as the electrophoresis tank, and connected to PowerEase (trademark) 90W Power Supply (Thermo Fisher Scientific), and electrophoresis was performed at a voltage of 200 V for 40 minutes. Thereafter, the gel was stained with GelCode (trademark) Blue Stain Reagent (Thermo Fisher Scientific), and the target protein bands were confirmed (Figure 2). As a result, it was confirmed that His-tagged CoVHH-N1 and His-tagged CoVHH-N2 were produced.

Example 3: Measurement of binding activity by surface plasmon resonance (SPR) resonance method

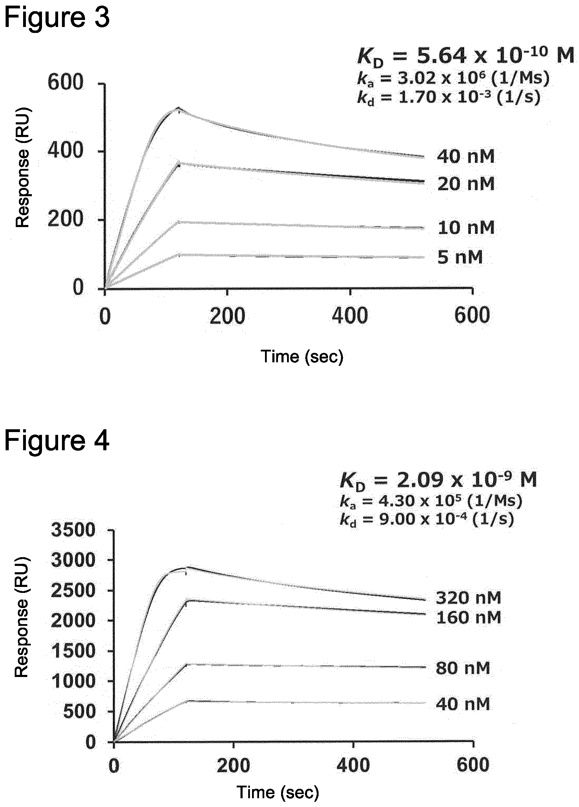

The binding activity of His-tagged CoVHH-N1 or His-tagged CoVHH-N2 immobilized on a Series S Sensor Chip NTA (Cytiva) to the N-protein was measured using Biacore (trademark) T200 (Cytiva). The measurements were performed in Wizard, Kinetics/Affinity mode. The temperature was set at 25°C. The running buffer used contained 10 mM HEPES, 300 mM NaCl, 50 µM EDTA, and 0.05% (v/v) Tween 20 (pH: 7.4). The measurement order for each run is as follows: 1) Immobilization of VHH: the flow rate was set to 10 µL/mL, 0.5 mM NiCl2 aqueous solution was added for 60 seconds, and a His-tagged VHH solution diluted with the running buffer was then added thereto for 60 seconds to reach an immobilization level of 1000 RU. 2) Measurement of binding activity: the flow rate was set to 30 µL/mL, and an association time was set to 120 seconds and a dissociation time was set to 400 seconds for interaction of SARS-CoV-2 Nucleocapsid-Fc fusion protein (Fc-tagged N-protein, InvivoGen) diluted with the running buffer. 3) Regeneration of sensor chip surface: the flow rate was set to 30 µL/mL, and 350 mM EDTA solution (pH: 8.0) was added for 60 seconds, thereby eluting the immobilized His-tagged VHH. Using Biacore (trademark) T200 Evaluation (software version 2.0) (Cytiva), analysis with a 1:1 binding model was preformed, and the binding activity was calculated (Figures 3 and 4). As a result, the equilibrium dissociation constant (KD) of His-tagged CoVHH-N1 for the N-protein was $5.64 \times 10^{-10}$ M (ka = $3.02 \times 10^6$ (1/Ms), kd = $1.70 \times 10^{-3}$ (1/s)). The equilibrium dissociation constant (KD) of His-tagged CoVHH-N2 for the N-protein was $2.09 \times 10^{-9}$ M (ka = $4.30 \times 10^5$ (1/Ms), kd = $9.00 \times 10^{-4}$ (1/s)).

Example 4: Evaluation of binding specificity by ELISA method

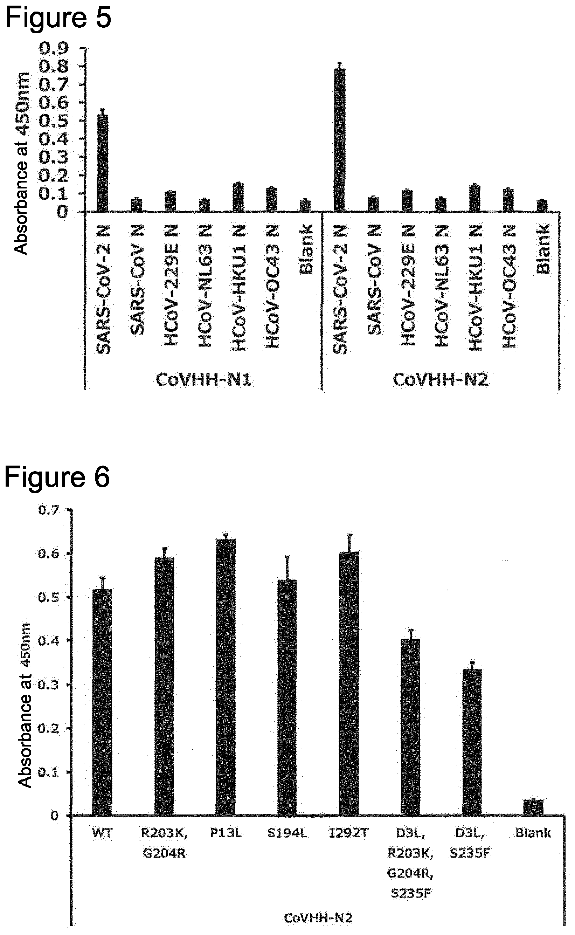

50 µL of 10 µg/mL SARS-CoV-2 (2019-nCoV) Nucleocapsid-His Recombinant Protein (His-tagged N-protein), Human coronavirus (HCoV-229E) Nucleoprotein/NP Protein (His Tag) (His-tagged N-protein), Human coronavirus (HCoV-NL63) Nucleoprotein/NP Protein (His Tag) (His-tagged N-protein), Human coronavirus (HCoV-HKU1) Nucleoprotein/NP Protein (His Tag) (His-tagged N-protein), and Human coronavirus (HCoV-OC43) Nucleoprotein/NP Protein (His Tag) (His-tagged N-protein) (all Sino Biological), and SARS-CoV nucleocapsid recombinant protein (His-SUMO tagged, Nter) (His-SUMO-tagged N-protein) (Arigo Biolaboratories) were added to each well of F96 Cert.Maxisorp Nunc-Immuno (trademark) Plate (Thermo Fisher Scientific), and after sealing, the resultant was allowed to stand at 4°C overnight. After removing the N-protein dispersion which was not adsorbed to the wells, 200 µL of PBST (0.05% (v/v) Tween 20-containing PBS) was added thereto and immediately removed. This washing operation was performed three times. 200 µL of Pierce (trademark) Protein-Free (PBS) Blocking Buffer (Thermo Fisher Scientific) was added thereto, and the mixture was incubated at room temperature for 1 hour. After removing the blocking buffer, 200 µL of PBST (0.05% (v/v) Tween 20-containing PBS) was added thereto and immediately removed. This washing operation was performed three times. 50 µL of 1 µg/mL His-tagged CoVHH-N1 or His-tagged CoVHH-N2/PBST (0.05% (v/v) Tween 20-containing PBS) was added to each well to which the N-protein was immobilized and incubated at room temperature for 1 hour. After removing the VHH antibody, 200 µL of PBST (0.05% (v/v) Tween 20-containing PBS) was added thereto and immediately removed. This washing operation was performed three times. The detection antibody used was Peroxidase-AffiniPure Goat Anti-Alpaca IgG (H+L) (min X Bov, Hu, Ms, Rb, Rat Sr Prot) (Jackson Immuno Research Laboratories) (1.0 mg/mL). The detection antibody was diluted to 1/5,000 with PBST (0.05% (v/v) Tween 20-containing PBS). 50 µL of the detection antibody was added to each well and incubated at room temperature for 1 hour. After removing the detection antibody, 200 µL of PBST (0.05% (v/v) Tween 20-containing PBS) was added thereto and immediately removed. This washing operation was performed three times. A chromogenic substrate was prepared by dissolving OPD Tablet (Thermo Fisher Scientific) in Stable Peroxide Substrate Buffer (Thermo Fisher Scientific). 100 $\mu$L of the chromogenic substrate was added to each well and incubated for 30 minutes in the dark, and then the absorbance at 450 nm was immediately measured using the Microplate Reader Infinite M1000 PRO (TECAN) (Figure 5). As a result, it was confirmed that CoVHH-N1 and CoVHH-N2 both specifically bound to the N-protein of SARS-CoV-2.

Example 5: Evaluation of binding activity for variant strain N-protein by ELISA method

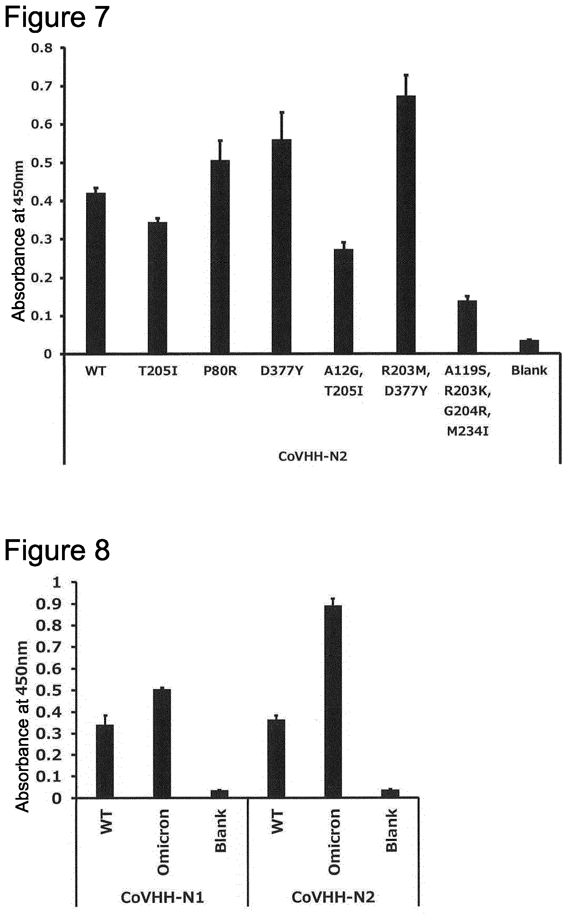

50 $\mu$l of 10 $\mu$g/ml SARS-CoV-2 (2019-nCoV) Nucleocapsid-His Recombinant Protein (His-tagged N-protein), SARS-CoV-2 (2019-nCoV) Nucleocapsid (R203K, G204R)-His Recombinant Protein, SARS-CoV-2 (2019-nCoV) Nucleocapsid (P13L)-His Recombinant Protein, SARS-CoV-2 (2019-nCoV) Nucleocapsid (S194L)-His Recombinant Protein, SARS-CoV-2 (2019-nCoV) Nucleocapsid (I292T)-His Recombinant Protein, SARS-CoV-2 (2019-nCoV) Nucleocapsid (D3L, R203K, G204R, S235F)-His Recombinant Protein, SARS-CoV-2 (2019-nCoV) Nucleocapsid (D3L, S235F)-His Recombinant Protein, SARS-CoV-2 (2019-nCoV) Nucleocapsid (T205I) Protein (His Tag), SARS-CoV-2 (2019-nCoV) Nucleocapsid (P80R) Protein (His Tag), SARS-CoV-2 (2019-nCoV) Nucleocapsid (D377Y) Protein (His Tag), SARS-CoV-2 Nucleocapsid (A12G, T205I) Protein (His Tag), SARS-CoV-2 Nucleocapsid (R203M, D377Y) Protein (His Tag), and SARS-CoV-2 Nucleocapsid (A119S, R203K, G204R, M234I) Protein (His Tag) (all Sino Biological) were added to each well of F96 Cert.Maxisorp Nunc-Immuno (trademark) Plate (Thermo Fisher Scientific), and after sealing, the resultant was allowed to stand at 4°C overnight. After removing the N-protein dispersion which was not adsorbed to the wells, 200 $\mu$l of PBST (0.05% (v/v) Tween 20-containing PBS) was added thereto and immediately removed. This washing operation was performed three times. 200 $\mu$l of Pierce (trademark) Protein-Free (PBS) Blocking Buffer (Thermo Fisher Scientific) was added thereto, and the mixture was incubated at room temperature for 1 hour. After removing the blocking buffer, 200 $\mu$l of PBST (0.05% (v/v) Tween 20-containing PBS) was added thereto and immediately removed. This washing operation was performed three times. 50 $\mu$l of 1 $\mu$g/mL CoVHH-N2 (His-tagged)/PBST (0.05% (v/v) Tween 20-containing PBS) was added to each well to which the N-protein was immobilized, and incubated at room temperature for 1 hour. After removing the VHH antibody, 200 $\mu$l of PBST (0.05% (v/v) Tween 20-containing PBS) was added thereto and immediately removed. This washing operation was performed three times. The detection antibody used was Peroxidase-AffiniPure Goat Anti-Alpaca IgG (H+L) (min X Bov, Hu, Ms, Rb, Rat Sr Prot) (Jackson Immuno Research Laboratories) (1.0 mg/mL). The detection antibody was diluted to 1/10,000 with PBST (0.05% (v/v) Tween 20-containing PBS). 50 $\mu$l of the detection antibody was added to each well and incubated at room temperature for 1 hour. After removing the detection antibody, 200 $\mu$l of PBST (0.05% (v/v) Tween 20-containing PBS) was added thereto and immediately removed. This washing operation was performed three times.
A chromogenic substrate was prepared by dissolving OPD Tablet (Thermo Fisher Scientific) in Stable Peroxide Substrate Buffer (Thermo Fisher Scientific). 100 $\mu$l of the chromogenic substrate was added to each well and incubated for 30 minutes in the dark, and then the absorbance at 450 nm was immediately measured using the Microplate Reader Infinite M1000 PRO (TECAN) (Figures 6 and 7).
As a result, it was confirmed that His-tagged CoVHH-N2 universally bound to the N-protein of SARS-CoV-2 variant strain.

Example 6: Evaluation of binding activity for omicron strain N-protein by ELISA method

50 $\mu$L of 10 $\mu$g/mL SARS-CoV-2 (2019-nCoV) Nucleocapsid-His Recombinant Protein (His-tagged N-protein) and SARS-CoV-2 B.1.529 (Omicron) Nucleocapsid Protein (His Tag) (all Sino Biological) were added to each well of F96 Cert.Maxisorp Nunc-Immuno (trademark) Plate (Thermo Fisher Scientific), and after sealing, the resultant was allowed to stand at 4°C overnight. After removing the N-protein dispersion which was not adsorbed to the wells, 200 $\mu$L of PBST (0.05% (v/v) Tween 20-containing PBS) was added thereto and immediately removed. This washing operation was performed three times. 200 $\mu$L of Pierce (trademark) Protein-Free (PBS) Blocking Buffer (Thermo Fisher Scientific) was added thereto, and the mixture was incubated at room temperature for 1 hour. After removing the blocking buffer, 200 $\mu$L of PBST (0.05% (v/v) Tween 20-containing PBS) was added thereto and immediately removed. This washing operation was performed three times. 50 $\mu$L of 1 $\mu$g/mL CoVHH-N1 (His-tagged) or CoVHH-N2 (His-tagged)/PBST (0.05% (v/v) Tween 20-containing PBS) was added to each well to which the N-protein was immobilized, and incubated at room temperature for 1 hour. After removing the VHH antibody, 200 $\mu$L of PBST (0.05% (v/v) Tween 20-containing PBS) was added thereto and immediately removed. This washing operation was performed three times. The detection antibody used was Peroxidase-AffiniPure Goat Anti-Alpaca IgG (H+L) (min X Bov, Hu, Ms, Rb, Rat Sr Prot) (Jackson Immuno Research Laboratories) (1.0 mg/mL). The detection antibody was diluted to 1/10,000 with PBST (0.05% (v/v) Tween 20-containing PBS). 50 $\mu$L of the detection antibody was added to each well and incubated at room temperature for 1 hour. After removing the detection antibody, 200 $\mu$L of PBST (0.05% (v/v) Tween 20-containing PBS) was added thereto and immediately removed. This washing operation was performed three times.
A chromogenic substrate was prepared by dissolving OPD Tablet (Thermo Fisher Scientific) in Stable Peroxide Substrate Buffer (Thermo Fisher Scientific). 100 μL of the chromogenic substrate was added to each well and incubated for 30 minutes in the dark, and then the absorbance at 450 nm was immediately measured using the Microplate Reader Infinite M1000 PRO (TECAN) (Figure 8).

As a result, it was confirmed that His-tagged CoVHH-N1 and His-tagged CoVHH-N2 both bound to the N-protein of SARS-CoV-2 omicron strain.

## Claims

1. An antibody that binds to SARS-CoV-2, having one or more structural domains comprising CDRs shown in the following (a) or (b):

   (a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or
   (b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

2. The antibody according to claim 1, which binds to N-protein.

3. The antibody according to claim 1 or 2, which is a VHH antibody, a heavy-chain antibody, or a VHH antibody multimer.

4. The antibody according to claim 3, wherein the VHH antibody multimer is a multimer in which the structural domains are linked to each other.

5. The antibody according to claim 3, wherein the VHH antibody multimer is a multimer in which one or more of the structural domains are linked to one or more structural domains with different antigen specificity from the former structural domains.

6. A nucleic acid encoding the antibody according to claim 1 or 2.

7. A method for detecting SARS-CoV-2 in a sample, comprising a step of contacting an antibody that binds to SARS-CoV-2 with a test sample, the antibody having one or more structural domains comprising CDRs shown in the following (a) or (b):

   (a) CDR1 consisting of the amino acid sequence of SEQ ID NO: 1 or an amino acid sequence obtained by substituting one amino acid with another amino acid in the amino acid sequence, CDR2 consisting of the amino acid sequence of SEQ ID NO: 2 or an amino acid sequence obtained by substituting one amino acid with another amino acid in the amino acid sequence, and CDR3 consisting of the amino acid sequence of SEQ ID NO: 3 or an amino acid sequence obtained by substituting one amino acid with another amino acid in the amino acid sequence; or
   (b) CDR1 consisting of the amino acid sequence of SEQ ID NO: 4 or an amino acid sequence obtained by substituting one amino acid with another amino acid in the amino acid sequence, CDR2 consisting of the amino acid sequence of SEQ ID NO: 5 or an amino acid sequence obtained by substituting one amino acid with another amino acid in the amino acid sequence, and CDR3 consisting of the amino acid sequence of SEQ ID NO: 6 or an amino acid sequence obtained by substituting one amino acid with another amino acid in the amino acid sequence.

8. A kit for detecting SARS-CoV-2, comprising the antibody according to any one of claims 1 to 5.

9. A medicament comprising the antibody according to any one of claims 1 to 5.

10. The medicament according to claim 9, for use in prevention or treatment of SARS-CoV-2 infection.

11. Use of the antibody according to any one of claims 1 to 5, for producing a medicament.

12. The use according to claim 11, wherein the medicament is a medicament for preventing or treating SARS-CoV-2 infection.

13. The antibody according to any one of claims 1 to 5, for use as a medicament.

14. The antibody according to claim 13, wherein the medicament is a medicament for preventing or treating SARS-CoV-2 infection.

15. A method for preventing or treating SARS-CoV-2 infection, comprising administering the antibody according to any one of claims 1 to 5 to a subject in need thereof.

# Figure 1

**Block 1 (residues 1–41)**

| | | | Sequence (1–41) |
|---|---|---|---|
| | CoVHH-N1 | 1 | A E V Q L V E S G G A V V Q P G G S L R L S C E A S G F P F T K F W M Y W V R R A 41 |
| | CoVHH-N1-1 | 1 | A E V Q L V E S G G A V V Q P G G S L R L S C E A S G F P F T [N] F W M Y W V R R A 41 |
| | CoVHH-N1-2 | 1 | A E V Q L V E S G G A V V Q P G G S L R L S C E A S G F P F T K [L] W M Y W V R R A 41 |
| | CoVHH-N1-3 | 1 | A E V Q L V E S G G A V V Q P G G S L R L S C E A S G F P [L] T K F W M Y W V R R A 41 |
| CDR1 variant | CoVHH-N1-4 | 1 | A E V Q L V E S G G A V V Q P G G S L R L S C E A S G [L] P F T K F W M Y W V R R A 41 |
| | CoVHH-N1-5 | 1 | A E V Q L V E S G G A V V Q P G G S L R L S C E A S G F P F T K F W [V] Y W V R R A 41 |
| | CoVHH-N1-6 | 1 | A E V Q L V E S G G A V V Q P G G S L R L S C E A S G F P F T K F W [R] Y W V R R A 41 |
| | CoVHH-N1-7 | 1 | A E V Q L V E S G G A V V Q P G G S L R L S C E A S [R] F P F T K F W M Y W V R R A 41 |
| | CoVHH-N1-8 | 1 | A E V Q L V E S G G A V V Q P G G S L R L S C E A S G F P F T K [C] W M Y W V R R A 41 |
| | CoVHH-N1-9 | 1 | A E V Q L V E S G G A V V Q P G G S L R L S C E A S G F P F T K F [C] M Y W V R R A 41 |
| CDR2 variant | CoVHH-N1-10 | 1 | A E V Q L V E S G G A V V Q P G G S L R L S C E A S G F P F T K F W M Y W V R R A 41 |
| | CoVHH-N1-11 | 1 | A E V Q L V E S G G A V V Q P G G S L R L S C E A S G F P F T K F W M Y W V R R A 41 |
| | CoVHH-N1-12 | 1 | A E V Q L V E S [E] G A V V Q P G G S L R L S C E A S G F P F T K F W M Y W V R R A 41 |
| | CoVHH-N1-13 | 1 | A E V Q L V E S G G A V V Q P G G S L R L S C E A S G F P F T K F W M Y W V R R A 41 |
| | CoVHH-N1-14 | 1 | A E V Q L V E S G G A V V Q P G G S L R L S C E A S G F P F T K F W M Y W V R R A 41 |
| | CoVHH-N1-15 | 1 | A E V Q L V E S G G A V V Q P G G S L R L S C E A S G F P F T K F W M Y W V R R A 41 |
| CDR3 variant | CoVHH-N1-16 | 1 | A E V Q L V E S G G A V V Q P G G S L R L S C E A S G F P F T K F W M Y W V R R A 41 |
| | CoVHH-N1-17 | 1 | A E V Q L V E S G G A V V Q P G G S L R L S C E A S G F P F T K F W M Y W V R R A 41 |
| | CoVHH-N1-18 | 1 | A E V Q L V E S G G A V V Q P G G S L R L S C [A] A S G F P F T K F W M Y W V R R A 41 |
| | CoVHH-N1-19 | 1 | A E V Q L V E S G G A [F] V Q P G G S L R L S C E A S G F P F T K F W M Y W V R R A 41 |
| | CoVHH-N1-20 | 1 | A E V Q L V E S G G A V V Q P G G S L R L S C E A S G F P F T K F W M Y W V R R A 41 |

CDR1

**Block 2 (residues 42–82)**

| | | | Sequence (42–82) |
|---|---|---|---|
| | CoVHH-N1 | 42 | P G K G L E W V A F I D T T G T I T N Y A S S V K G R F T I S R D D A R N M V Y L 82 |
| | CoVHH-N1-1 | 42 | P G K G L E W V A F I D T T G T I T N Y A S S V K G R F T I S R D D A R N M V Y L 82 |
| | CoVHH-N1-2 | 42 | P G K G L E W V A F I D T T G T I T N Y A S S V K G R F T I S R D D A R N M V Y L 82 |
| | CoVHH-N1-3 | 42 | P G K G L E W V A F I D T T G T I T N Y A S S V K G R F T I S R D D A R N M V Y L 82 |
| CDR1 variant | CoVHH-N1-4 | 42 | P G K G L E W V A F I D T T G T I T N Y A S S V K G R F T I S R D D A R N M V Y L 82 |
| | CoVHH-N1-5 | 42 | P G K G L E W V A F I D T T G T I T N Y A S S V K G R F T I S R D D A R N M V Y L 82 |
| | CoVHH-N1-6 | 42 | P G K G L E W V A F I D T T G T I T N Y A S S V K G R F T I S R D D A R N M V Y L 82 |
| | CoVHH-N1-7 | 42 | P G K G L E W V A F I D T T G T I T N Y A S S V K G R F T I S R D D A R N M V Y L 82 |
| | CoVHH-N1-8 | 42 | P G K G L E W V A F I D T T G T I T N Y A S S V K G R F T I S R D D A R N M V Y L 82 |
| | CoVHH-N1-9 | 42 | P G K G L E W V A F I D T T G T I T N Y A S S V K G R F T I S R D D A R N M V Y L 82 |
| CDR2 variant | CoVHH-N1-10 | 42 | P G K G L E W V A [L] I D T T G T I T N Y A S S V K G R F T I S R D D A R N M V Y L 82 |
| | CoVHH-N1-11 | 42 | P G K G L E W V A F I D T T G T I T [D] Y A S S V K G R F T I S R D D A R N M V Y L 82 |
| | CoVHH-N1-12 | 42 | P G K G L E W V A F I D T T G T I T [D] Y A S S V K G R F T I S R D D A R N M V Y L 82 |
| | CoVHH-N1-13 | 42 | P G K G L E W V A F I D T T G T I T N Y A S S V K G R F T I S R D D A R N M V Y L 82 |
| | CoVHH-N1-14 | 42 | P G K G L E W V A F I D T T G T I T N Y A S S V K G R F T I S R D D A R N M V Y L 82 |
| | CoVHH-N1-15 | 42 | P G K G L E W V A F I D T T G T I T N Y A S S V K G R F T I S R D D A R N M V Y L 82 |
| CDR3 variant | CoVHH-N1-16 | 42 | P G K G L E W V A F I D T T G T I T N Y A S S V K G R F T I S R D D A R N M V Y L 82 |
| | CoVHH-N1-17 | 42 | P G K G L E W V A F I D T T G T I T N Y A S S V K G R F T I S R D D A R N M V Y L 82 |
| | CoVHH-N1-18 | 42 | P G K G L E W V A F I D T T G T I T N Y A S S V K G R F T I S R D D A R N M V Y L 82 |
| | CoVHH-N1-19 | 42 | P G K G L E W V A F I D T T G T I T N Y A S S V K G R F T I S R D D A R N M V Y L 82 |
| | CoVHH-N1-20 | 42 | P G K G L E W V A F I D T T G T I T N Y A S S V K G R F T I S R D D A R N M V Y L 82 |

CDR2

**Block 3 (residues 83–123)**

| | | | Sequence (83–123) |
|---|---|---|---|
| | CoVHH-N1 | 83 | Q M N D L R P E D T A I Y Y C A T G P P G S T W V V D F H S W G Q G T Q V T V S S 123 |
| | CoVHH-N1-1 | 83 | Q M N D L R P E D T A I Y Y C A T G P P G S T W V V D F H S W G Q G T Q V T V S S 123 |
| | CoVHH-N1-2 | 83 | Q M N D L R P E D T A I Y Y C A T G P P G S T W V V D F H S W G Q G T Q V T V S S 123 |
| | CoVHH-N1-3 | 83 | Q M N D L R P E D T A I Y Y C A T G P P G S T W V V D F H S W G Q G T Q V T V S S 123 |
| CDR1 variant | CoVHH-N1-4 | 83 | Q M N D L R P E D T A I Y Y C A T G P P G S T W V V D F H S W G Q G T Q V T V S S 123 |
| | CoVHH-N1-5 | 83 | Q M N D L R P E D T A I Y Y C A T G P P G S T W V V D F H S W G Q G T Q V T V S S 123 |
| | CoVHH-N1-6 | 83 | Q M N D L R P E D T A I Y Y C A T G P P G S T W V V D F H S W G Q G T Q V T V S S 123 |
| | CoVHH-N1-7 | 83 | Q M N D L R P E D T A I Y Y C A T G P P G S T W V V D F H S W G Q G T Q V T V S S 123 |
| | CoVHH-N1-8 | 83 | Q M N D L R P E D T A I Y Y C A T G P P G S T W V V D F H S W G Q G T Q V T V S S 123 |
| | CoVHH-N1-9 | 83 | Q M N D L R P E D T A I Y Y C A T G P P G S T W V V D F H S W G Q G T Q V T V S S 123 |
| CDR2 variant | CoVHH-N1-10 | 83 | Q M N D L R P E D T A I Y Y C A T G P P G S T W V V D F H S W G Q G T Q V T V S S 123 |
| | CoVHH-N1-11 | 83 | Q M N D L R P E D T A I Y Y C A T G P P G S T W V V D F H S W G Q G T Q V T V S S 123 |
| | CoVHH-N1-12 | 83 | Q M N D L R P E D T A I Y Y C A T G P P G S T W V [V] D F H S W G Q G T Q V T V S S 123 |
| | CoVHH-N1-13 | 83 | Q M N D L R P E D T A I Y Y C A T G P P G S T W V [I] D F H S W G Q G T Q V T V S S 123 |
| | CoVHH-N1-14 | 83 | Q M N D L R P E D T A I Y Y C A T G P P G S [A] W V V D F H S W G Q G T Q V T V S S 123 |
| | CoVHH-N1-15 | 83 | Q M N D L R P E D T A I Y Y C A T G P P G S T W V V D F [N] S W G Q G T Q V T V S S 123 |
| CDR3 variant | CoVHH-N1-16 | 83 | Q M N D L R P E D T A I Y Y C A T [C] P P G S T W V V D F H S W G Q G T Q V T V S S 123 |
| | CoVHH-N1-17 | 83 | Q M N D L R P E D T A I Y Y C A T G P P G S T W V V D F [D] S W G Q G T Q V T V S S 123 |
| | CoVHH-N1-18 | 83 | Q M N D L R P E D T A I Y Y C A T G P P G S [A] W V V D F H S W G Q G T Q V T V S S 123 |
| | CoVHH-N1-19 | 83 | Q M N D L R P E D T A I Y Y C A T G P P G S T W V V D F [N] S W G Q G T Q V T V S S 123 |
| | CoVHH-N1-20 | 83 | Q M N D L [S] P E D T A I Y Y C A T G P P G S [P] W V V D F H S W G Q G T Q V T V S S 123 |

CDR3

# Figure 2

## Figure 3

$K_D = 5.64 \times 10^{-10}$ M
$k_a = 3.02 \times 10^6$ (1/Ms)
$k_d = 1.70 \times 10^{-3}$ (1/s)

## Figure 4

$K_D = 2.09 \times 10^{-9}$ M
$k_a = 4.30 \times 10^5$ (1/Ms)
$k_d = 9.00 \times 10^{-4}$ (1/s)

## Figure 5

## Figure 6

# Figure 7

# Figure 8

## INTERNATIONAL SEARCH REPORT

| International application No. |
|---|
| **PCT/JP2022/006996** |

**A. CLASSIFICATION OF SUBJECT MATTER**

*C12N 15/13*(2006.01)i; *A61K 39/395*(2006.01)i; *A61P 31/14*(2006.01)i; *A61P 43/00*(2006.01)i; *C07K 16/10*(2006.01)i; *C07K 19/00*(2006.01)i; *G01N 33/569*(2006.01)i
FI:  C12N15/13 ZNA; C07K19/00; A61K39/395 N; A61P31/14; A61P43/00 105; G01N33/569 L; C07K16/10

According to International Patent Classification (IPC) or to both national classification and IPC

**B. FIELDS SEARCHED**

Minimum documentation searched (classification system followed by classification symbols)

C12N15/13; A61K39/395; A61P31/14; A61P43/00; C07K16/10; C07K19/00; G01N33/569

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); GenBank/EMBL/DDBJ/GeneSeq; UniProt/GeneSeq; PubMed

**C. DOCUMENTS CONSIDERED TO BE RELEVANT**

| Category* | Citation of document, with indication, where appropriate, of the relevant passages | Relevant to claim No. |
|---|---|---|
| X | CN 112028993 A (CUSABIO BIOTECH CO., LTD.) 04 December 2020 (2020-12-04) abstract, paragraph [0004], claim 4 | 1-8 |
| Y | abstract, paragraph [0004], claim 4 | 9-15 |
| Y | CN 111153991 A (BEIJING BIOSYNTHESIS BIOTECHNOLOGY CO., LTD.) 15 May 2020 (2020-05-15) abstract, claim 7 | 9-15 |
| P, A | WO 2021/181994 A1 (DENKA CO., LTD.) 16 September 2021 (2021-09-16) entire text | 1-15 |
| P, A | YE Q., LU S., CORBETT KD. Structural Basis for SARS-CoV-2 Nucleocapsid Protein Recognition by Single-Domain Antibodies., Front. Immunol., 26 July 2021, doi: 10.3389/fimmu.2021.719037. entire text | 1-15 |

☑ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 April 2022** | **19 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/006996**

| C. | DOCUMENTS CONSIDERED TO BE RELEVANT | |
|---|---|---|
| Category* | Citation of document, with indication, where appropriate, of the relevant passages | Relevant to claim No. |
| P, A | GRANSAGNE, M. et al. Development of a highly specific and sensitive VHH-based sandwich immunoassay for the detection of the SARS-CoV-2 nucleoprotein., J. Biol. Chem., January 2022, doi: 10.1016/j.jbc.2021.101290.<br>    entire text | 1-15 |

Form PCT/ISA/210 (second sheet) (January 2015)

**INTERNATIONAL SEARCH REPORT**

| International application No. |
|---|
| **PCT/JP2022/006996** |

| Box No. I      Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/006996**

| Patent document cited in search report | | | Publication date (day/month/year) | Patent family member(s) | Publication date (day/month/year) |
|---|---|---|---|---|---|
| CN | 112028993 | A | 04 December 2020 | (Family: none) | |
| CN | 111153991 | A | 15 May 2020 | (Family: none) | |
| WO | 2021/181994 | A1 | 16 September 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014158430 A
- JP 2006174707 A

- JP 4336082 B

**Non-patent literature cited in the description**

- A pneumonia outbreak associated with a new coronavirus of probable bat origin. *Nature,* March 2020, vol. 579 (7798), 270-273
- A new coronavirus associated with human respiratory disease in China. *Nature,* March 2020, vol. 579 (7798), 265-269
- The Coronavirus Nucleocapsid Is a Multifunctional Protein. *Viruses,* August 2014, vol. 6 (8), 2991-3018

- The SARS-CoV nucleocapsid protein: A protein with multifarious activities. *Infection, Genetics and Evolution,* July 2008, vol. 8, 397-405
- Diagnosis of Severe Acute Respiratory Syndrome (SARS) by Detection of SARS Coronavirus Nucleocapsid Antibodies in an Antigen-Capturing Enzyme-Linked Immunosorbent Assay. *Journal of Clinical Microbiology.,* December 2003, vol. 41 (12), 5781-5782
- The Therapeutic Potential of Nanobodies. *BioDrugs.,* November 2019, vol. 34, 11-26